# EUROPEAN PATENT APPLICATION

(11) **EP 2 476 753 A1**
(43) Date of publication of application: **18.07.2012**
(21) Application number: 10815279.4
(22) Date of filing: 30.08.2010
(51) Int. Cl.: C12N 15/09, A61K 47/42, C07K 17/00, C07K 14/025

(54) **CONSTRUCT COATED WITH VIRUS COAT-CONSTITUTING PROTEIN AND METHOD FOR PRODUCING SAME**

(30) Priority: 09.09.2009 JP 2009207632
(71) Applicant: Tokyo Institute of Technology, Tokyo 152-8550 (JP)
(72) Inventor: HANDA, Hiroshi, Yokohama-shi Kanagawa 226-8503 (JP); DOI, Koji, Yokohama-shi Kanagawa 226-8503 (JP); ENOMOTO, Teruya, Yokohama-shi Kanagawa 226-8503 (JP); KAWANO, Masaaki, Yokohama-shi Kanagawa 226-8503 (JP)
(74) Representative: Ford, Hazel
(86) International application number: PCT/JP2010/064667
(87) International publication number: WO 2011/030682

(57) **Abstract**

Disclosed is a construct comprising a virus coat protein usable as a carrier for drug delivery adaptable to various drugs. Specifically disclosed is a construct coated with a virus coat protein, comprising a virus coat protein and a construct coated therewith, wherein the virus coat protein is attached to the surface of the construct to form a layer of the protein thereon.

## Description

### Technical Field

The present invention relates to a construct coated with a virus coat protein and a method for producing the same. The construct coated with a virus coat protein of the present invention is useful as a carrier for drug delivery or gene therapy.

### Background Art

A drug delivery system is necessary for controlling in vivo drug distribution and optimizing drug dosage without causing side effects. In the drug delivery system, a carrier for delivering a drug is important. The carrier for drug delivery has conventionally used a cationic lipid (for example, Lipofectin (TM) reagent), a cationic polymer (for example, polyethylenimine), a ceramic material (for example, calcium phosphate), or the like. Examples of the carrier for an anticancer agent approved by the FDA include Doxyl (trademark) in which doxorubicin is encapsulated using MPEG-DSPE-modified liposomes and Abraxane (trademark) in which paclitaxel is bound to human serum albumin. These carriers have no active targeting capability against cancer; however, it can enhance the action of an anticancer agent because of having a characteristic of tending to accumulate in a structure typical of cancer tissue, called an EPR (enhanced permeability and retention) effect.

The present inventors have recently developed a carrier for drug delivery using a virus coat protein and have previously filed patent applications (Patent Literatures 1 and 2). The virus coat protein has excellent properties as a carrier for drug delivery, including 1) the capability to selectively bind to host cells via a cell membrane receptor (cell tropism), 2) the capability to enter into host cells through endocytosis (intracellular transport capability), 3) the capability to form a nano-structure by self-assembly (nano-capsules), and 4) the activity of packaging a virus genome (encapsulating activity).

### Citation List

### Patent Literature

Patent Literature 1: International Publication No. WO 2006/004173
Patent Literature 2: International Publication No. WO 2007/116808

### Summary of Invention

### Technical Problem

To establish a drug delivery system for various drugs, a carrier is necessary which can deliver these drugs to affected sites irrespective of their size and shape. However, the carriers described in Patent Literatures 1 and 2 above each reconstitute a virus coat protein to a virus particle and package a drug in the reconstituted virus particle; thus, the size of the drug is limited to such a large size that it can not be packaged in the virus particle.

Made under such a technical background, the present invention has an object of providing a carrier for drug delivery adaptable to various drugs.

### Solution to Problem

As the result of intensive studies for solving the above-described problems, the present inventors have found that the virus coat protein can be non-specifically attached to the surface of a construct, which has such a large size that it can not be packaged in virus particles, to form a layer of the virus coat protein thereon, which coats the construct.

Patent Literatures 1 and 2 describe that a virus coat protein is reconstituted to a virus particle (for example, lines 3 to 5 of page 2 of Patent Literature 1 and lines 22 to 26 of page 5 of Patent Literature 2) and also describe that a drug or the like to be delivered is packaged in the virus particle (for example, lines 8 to 10 of page 2 of Patent Literature 1 and lines 26 to 28 of page 5 of Patent Literature 2).

The above-described construct coated with a virus coat protein found by the present inventor has a structure different from that of a virus particle; thus, this construct is different from the virus particle in which a drug or the like is packaged described in Patent Literatures 1 and 2.

At the time of the present application, it was considered that an assembly of virus coat protein molecules had a followable structure fixed by salt concentration, pH, and the like and could not be freely changed in the size and shape thereof. For example, Kanesashi et al. report that the pentamer of VP1 (the coat protein of SV40 virus) forms 1) nanocapsules having particle diameters of 20 nm, 32 nm and 45 nm and a tube-like construct under conditions of high salt concentrations including 1 M NaCl and 2 mM CaCl₂, 2) only uniform nanocapsules having a particle diameter of 20 nm under conditions of only 1 M NaCl without CaCl₂, 3) uniform nanocapsules having a particle diameter of 45 nm under conditions of 2 M (NH₄)₂SO₄ and 2 mM CaCl₂, and 4) only a tube-like construct under conditions of pH 5.0 in the presence of 150 mM NaCl and 2 mM CaCl₂ (Kanesashi et al., J. Gen. Virol., 2003, 1899-1905).

Thus, it has been totally unexpected that an assembly of virus coat protein molecules can enclose each of constructs having various sizes and shapes to match the size and shape thereof.

The present invention has been completed based on the above findings.

Thus, the present invention provides the following (1) to (15).
(1) A construct coated with a virus coat protein, comprising a virus coat protein and a construct coated therewith, wherein the virus coat protein is attached to the surface of the construct to form a layer of the protein thereon.
(2) The construct coated with a virus coat protein according to (1), wherein the virus coat protein is non-specifically attached to the surface of the construct.
(3) The construct coated with a virus coat protein according to (1) or (2), wherein the construct is a construct having such a large size that it can not be packaged in a virus particle.
(4) The construct coated with a virus coat protein according to (1) or (2), wherein the construct has a particle diameter of 30 nm or more.
(5) The construct coated with a virus coat protein according to any of (1) to (4), wherein the virus coat protein is a coat protein of SV40 virus.
(6) A method for producing a construct coated with a virus coat protein, comprising incubating the virus coat protein and the construct at 15 to 30°C and pH 5 to pH 10 in the presence of a 100 mM to 500 mM monovalent cation and a 2 µM to 50 mM divalent cation, wherein the construct is a construct having such a large size that it can not be packaged in a virus particle.
(7) The method for producing a construct coated with a virus coat protein according to (6), wherein the construct has a particle diameter of 30 nm or more.
(8) The method for producing a construct coated with a virus coat protein according to (6) or (7), wherein the virus coat protein is a coat protein of SV40 virus.
(9) A method for treating a disease in an animal, comprising administering a drug to the animal to deliver the drug to an affected site of the animal, wherein the drug to be administered is a drug to the surface of which a virus coat protein is attached to form a layer of the protein thereon.
(10) The method for treating a disease according to (9), wherein the animal is a non-human animal.
(11) The method for treating a disease according to (9) or (10), wherein a substance having affinity for the affected site is added to the virus coat protein.
(12) The method for treating a disease according to any of (9) to (11), wherein the virus coat protein is non-specifically attached to the surface of the drug.
(13) The method for treating a disease according to any of (9) to (12), wherein the drug is a drug which is large enough not to be packaged in a virus particle.
(14) The method for treating a disease according to any of (9) to (13), wherein the drug has a particle diameter of 30 nm or more.
(15) The method for treating a disease according to any of (9) to (14), wherein the virus coat protein is a coat protein of SV40 virus.

### Advantageous Effects of Invention

The present invention enables the coating of any of various drugs with a virus coat protein irrespective of their size and shape, enabling the easy delivery of the drug to an affected site.

A plurality of different drugs can be coated with an identical virus coat protein to add a modifying group to the plurality of drugs at a time.

### Brief Description of Drawings

[Figure 1] Figure 1 is a pair of an electron photomicrograph of polystyrene beads coated with a virus coat protein (bottom) and an electron photomicrograph of polystyrene beads not coated with a virus coat protein (top).
[Figure 2] Figure 2 is a pair of an electron photomicrograph of ferrite particles coated with a virus coat protein (bottom) and an electron photomicrograph of ferrite particles not coated with a virus coat protein (top).
[Figure 3] Figure 3 is a pair of an electron photomicrograph of a DOX-containing PLA spherical construct coated with a virus coat protein (bottom) and an electron photomicrograph of a DOX-containing PLA spherical construct not coated with a virus coat protein (top).
[Figure 4] Figure 4 is a pair of an electron photomicrograph of a siRNA-containing PLGA spherical construct coated with a virus coat protein (bottom) and an electron photomicrograph of a siRNA-containing PLGA spherical construct not coated with a virus coat protein (top).
[Figure 5] Figure 5 is a pair of an electron photomicrograph of polystyrene beads stained with colloidal gold when an anti-VP1 antibody is added (bottom) and an electron photomicrograph of polystyrene beads stained with colloidal gold when an anti-VP1 antibody is not added (top). The black point indicated by an arrow is the colloidal gold.
[Figure 6] Figure 6 is a pair of an electron photomicrograph of silica beads stained with colloidal gold when an anti-VP1 antibody is added (bottom) and an electron photomicrograph of silica beads stained with colloidal gold when an anti-VP1 antibody is not added (top).

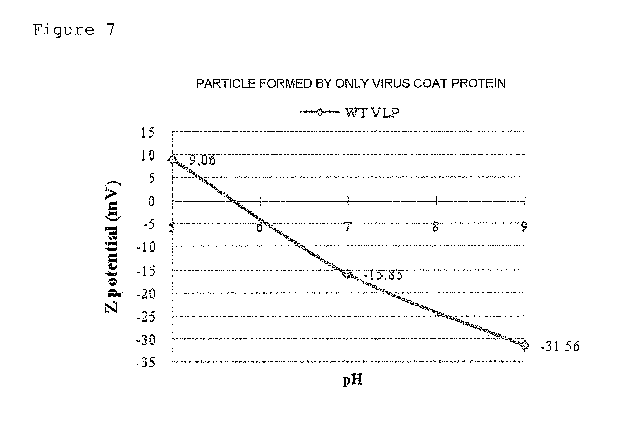

[Figure 7] Figure 7 is a graph showing the ζ potential of particles formed by only a virus coat protein.
[Figure 8] Figure 8 is a series of graphs showing the ζ potential of a negatively charged construct coated with a virus coat protein and a negatively charged construct not coated therewith. The top graph represents a case where polystyrene beads having an average particle diameter of 100 nm are used; the intermediate graph represents a case where polystyrene beads having an average particle diameter of 200 nm are used; and the bottom graph represents a case where polystyrene beads having an average particle diameter of 500 nm are used.
[Figure 9] Figure 9 is a graph showing the ζ potential of a positively charged construct (silica beads) coated with a virus coat protein and a positively charged construct not coated therewith.
[Figure 10] Figure 10 is a pair of an electron photomicrograph of a siRNA-containing liposome coated with a virus coat protein (bottom) and an electron photomicrograph of a siRNA-containing liposome not coated with a virus coat protein (top).
[Figure 11] Figure 11 is a graph showing the capabilities of various cells to ingest ferrite-containing virus-like particles on which EGF is fixed and ferrite-containing virus-like particles on which EGF is not fixed. The coordinate of the graph shows the content of iron in cells.
[Figure 12] Figure 12 is a pair of T2-weighed images of MRI in a mouse before and after administering ferrite-containing virus-like particles.

### Description of Embodiments

The present invention will be described below in detail.

The construct coated with a virus coat protein of the present invention comprises a virus coat protein and a construct coated therewith, wherein the virus coat protein is attached to the surface of the construct to form a layer of the protein thereon. Preferably, the virus coat protein is non-specifically attached to the surface of the construct.

### (1) Virus Coat Protein

The type of a virus supplying the virus coat protein is not particularly limited, and various proteins from various viruses may each be used in the coated construct of the present invention. Specific viruses to which the present invention can be applied can include a virus belonging to the family papovavirus such as SV40 virus, JCV virus, or BKV virus; and a virus such as a hepadnavirus (hepatitis B virus or the like), an adenovirus, a flavivirus (Japanese encephalitis virus or the like), a herpesvirus (herpes simplex virus, varicella-zoster virus, cytomegalovirus, EB virus, or the like), a poxvirus, a parvovirus (adeno-associated virus or the like), an orthomyxovirus (influenza virus or the like), a rhabdovirus (rabies virus or the like), a retrovirus (acquired immunodeficiency syndrome virus or the like), a hepatitis C virus, a lentivirus, a herpesvirus, a bacteriophage, an influenza virus, Sendai virus, a vaccinia virus, or a baculovirus. Especially, a virus having an icosahedral structure is preferable.

Affinity and adsorption specificity for cells of a host (also including a human subject receiving the administration of the coated construct of the present invention) vary depending on the virus; thus, the type of a virus is preferably selected in consideration of tropic characteristics of a necessary coated construct to use the protein thereof. This can provide a specific targeting capability. The accumulation of a conventional carrier on target cancer cells and tissue is due to the EPR effect; thus, the conventional carrier has had no specific targeting capability.

When the virus coat protein is used in a preparation applied to a living body, the immediate problem is the safety thereof. Some viruses themselves have pathogenicity or cytotoxic or antigenic structural proteins. In view of this, a virus coat protein from a virus belonging to the family papovavirus, particularly SV40 virus, JCV virus, or BKV virus, can be said to be preferable. These viruses cause almost no pathological symptoms due thereto when they infect hosts, and are considered to be harmless or have extremely low pathogenicity to humans. Papovaviruses capable of infecting a primate, especially SV40 virus, having high safety to humans and high structural stability, have the advantage of showing no antigenicity when they are administered to humans. On the other hand, because JCV virus is a virus capable of infecting a site of the brain, the protein thereof has the characteristic of being capable of passing through the blood-brain barrier; however, it has antigenicity. In the coated construct of the present invention, the virus coat protein is particularly preferably one derived from the family papovavirus.

Some viruses each have a plurality of virus coat proteins. For example, SV40 virus has the three proteins: VP1 as the major virus coat protein and VP2 and VP3 as minor virus coat proteins. In the present invention, it is preferable to use only the major virus coat protein VP1.

The virus coat protein may be used by purifying a native protein obtained from a naturally-occurring virus; however, a mutant or altered product of the virus coat protein, subjected to necessary modification, may also be preferably used. Alternatively, the native virus coat protein may also be used together with the mutant or altered product thereof. The reason for using such a mutant or altered product is that the effect can be expected of reducing the antigenicity of a virus particle-like construct or making the protein less recognizable by immune surveillance (into the so-called "stealthified" state). In view of providing a targeting function, the need also occurs for enhancing affinity for a particular organ, tissue, or cell. Alternatively, the need also exists for increasing the stability of the coated construct. In addition, it is necessary to comply with the requirements of forming parts capable of adding various functions to the protein surface. The appropriate modification of a protein molecule can comply with requirements from such various points of view.

The hydrophilic tendency of a virus coat protein can be enhanced to increase the stability thereof in the blood and maintain the blood concentration thereof over a long period of time. To improve retentivity in the blood, a polyethylene glycol (PEG) or polyalkylene oxide chain can be introduced into the protein surface to increase hydrophilicity. The length and introduction ratio of the oxyethylene unit can be properly changed to regulate the function thereof. The PEG is preferably a polyethylene glycol having 10 to 3,500 oxyethylene units.

A "functional substance" may further be added as a functional group to the end of the polyethylene glycol or polyalkylene oxide chain introduced into the surface of a virus coat protein. In addition to the effect of the introduction of the polyalkylene oxide chain, the coated construct on which the polyalkylene oxide chain having the "functional substance" bound to the end is immobilized sufficiently exerts the function of the "functional substance", for example, an effect such as tropism for a particular organ or tropism for a particular tissue as a "recognition element", without being obstructed by the polyalkylene oxide chain.

A drug molecule, a reporter marker (for example, a chromogenic substance, an enzyme, or a radioactive marker), or an affinity ligand (for example, an antibody or antibody fragment, a partner counterpart capable of specific binding (specifically, biotin or streptavidin), an oligopeptide, an oligonucleotide, or an oligosaccharide) may also be coupled as the "functional substance" to the virus coat protein to functionalize the surface of the coated construct, actually the surface of the virus coat protein. Direct coupling takes place through a functional group of protein, for example, a functional group rich in reactivity such as an amino group, an oxycarbonylimidazole group, or an N-hydroxysuccinic acid imide group.

Examples of the affinity ligand include a monoclonal antibody, a polyclonal antibody, an antibody fragment, a nucleic acid, an oligonucleotide, a protein, an oligopeptide, a polysaccharide, a saccharide, a nucleic acid molecule encoding a peptide, a lectin, a cell-adhesion factor, an antigen, a drug, and other ligands.

As described above, the virus coat protein may be a mutant or altered product functionally equivalent to a native virus coat protein. Specifically, it may be a mutant or altered product which retains the ability to coat the construct and in which the tropism for a target tissue or cell of the native virus coat protein is altered. The mutant or altered product in this case is obtained using a mutation induction method, a chemical modification method, or a gene recombination technique. The mutant or altered product of the virus coat protein is a protein in which at least one, preferably one or several, more preferably 1 to 24, still more preferably 1 to 15 amino acid residues are deleted, added, or replaced by other amino acid residues in the amino acid sequence forming the virus coat protein. The mutant or altered product is also one having the ability to coat the construct.

As used herein, "deleted" refers to the fact that at least one amino residue is lost from the polypeptide chain of a native protein. Conversely, "added" refers to the fact that at least one amino acid is inserted into the polypeptide chain; amino acids may be inserted in one position in the form of a peptide. "Substituted" refers to the fact that an amino acid residue in a particular site is replaced by another type of amino acid.

The term "functionally equivalent" corresponds to the fact that the amino acid sequence is modified by the deletion, substitution, addition and/or insertion of one or a plurality of amino acids or has the side chain in an amino acid residue chemically modified by phosphorylation or dephosphorylation, glucosylation or deglucosylation, or the like, but despite that, maintains an effect or activity substantially equivalent to that of a native virus coat protein. Such a functionally equivalent mutant can occur as a result of natural biological mutation; however, it may be prepared by the induction of mutation at a specified site or the induction of mutation at an unspecified site. In addition, the altered product may be produced using a known technique such as chemical modification, enzymatic cleavage and/or ligation reaction. It may also be prepared by a recombination means in which an appropriate nucleic acid molecule having an expression control sequence operably linked is expressed in host cells or in which a recombinant DNA cloning vector containing such a recombinant DNA molecule is expressed, or by chemical or biochemical synthesis (extracellular).

The mutant of a virus coat protein, the artificially partially or totally synthesized virus coat protein from a native protein, or the altered product obtained by altering a protein of biological origin may be one having a function not present in the protein of the viral origin as described above or having an inappropriate property, e.g., antigenicity, reduced.

Some mutants or altered products of a virus coat protein lose the ability to coat the construct or have increased antigenicity. Such mutants or altered products cannot be used in the coated construct of the present invention. Thus, the mutant or altered product of a virus coat protein is pursued by the need for preliminarily checking whether they exhibit such a disadvantageous property or not.

### (2) Construct

The type of the construct is not particularly limited, and examples thereof can include constructs comprising polymers such as a polystyrene bead, a silica bead, a metal compound particle, PLA (poly-lactic acid) and PLGA (co-poly lactic acid/glycolic acid) and a construct such as a liposome.

The shape of the construct is also not particularly limited, and may be, for example, a spherical, cubical, membranal, or fibrous shape.

The construct may have a positive or negative charge, and may also have no charge.

When the construct is small, the virus coat protein forms a virus particle and the construct is packaged in the particle; thus, the construct is preferably one having such a large size that it can not be packaged in the virus particle. The "such a large size that it can not be packaged in the virus particle" varies depending on the type and the like of the virus; thus, it may be determined according to the virus coat protein used. In the case of SV40 virus, the construct having particle diameter of 30 nm or more is typically large enough not to be packaged in the virus particle. Thus, the average particle diameter of the construct is preferably set to 30 nm or more, more preferably 45 nm or more. The average particle diameter of the construct may exceed 50 nm or exceed 100 nm. In the case of a virus other than SV40 virus, the average particle diameter may be similar to the value described above.

The upper limit of the average particle diameter of the construct is not particularly limited; however, the average particle diameter is preferably 500 nm or less because too large particle diameter eliminates practical use. As used herein, "particle diameter" is the diameter of a sphere if the construct is spherical; if the construct has a non-spherical shape such as a cube, a smallest sphere capable of packaging the construct therein is set and the diameter of the sphere is defined as the particle diameter. If it is a fibrous substance capable of being folded like DNA, a smallest sphere in which the construct in a folded state can be packaged is set and the diameter of the sphere is defined as the particle diameter.

### (3) Production Method

The method for producing the construct coated with a virus coat protein of the present invention is a method which involves incubating the virus coat protein and the construct at pH 5 to pH 10 and 15 to 30°C in the presence of a 100 mM to 500 mM monovalent cation and a 2 µM to 50 mM divalent cation, wherein the construct has such a large size that it can not be packaged in a virus particle.

Specifically, it comprises the steps of (I) mixing the construct while well stirring in the virus coat protein purified using an existing protein purification technique and (II) incubating the mixture at pH 5 to pH 10 and 15 to 30°C in the presence of a 100 mM to 500 mM monovalent cation and a 2 µM to 50 mM divalent cation.

After the step (I) and before the step (II), a buffer solution containing a salt (e.g., NaCl or CaCl₂), a chelator (e.g., EDTA or EGTA), and further, if necessary, a reducing agent (e.g., DTT) and the like may be added, followed by incubating the mixed solution at 4°C for a predetermined period of time.

Examples of one preferable embodiment of the step (II) include dialyzing the solution with a solution of pH 5 to 10 containing a 100 mM to 500 mM monovalent cation and a 2 µM to 50 mM divalent cation at 15 to 30°C for 15 to 20 hours, preferably 16 hours.

Examples of the monovalent cation include sodium ion, potassium ion, and ammonium ion; preferred is sodium ion. Examples of the divalent cation include magnesium ion and calcium ion; preferred is calcium ion. In addition, the concentration of the monovalent cation is preferably 100 to 500 mM, more preferably 100 to 200 mM, still more preferably 140 to 160 mM, most preferably 150 mM. The concentration of the divalent cation is preferably 1 to 5 mM, more preferably 2 mM.

According to the production method of the present invention, the ratio of the virus coat protein weight to the construct volume is preferably 4 µg or more/m³, more preferably 4 µg/m³.

### (4) Aspect of Use

The coated construct of the present invention can be used in various applications. Some specific examples thereof are given below.

### (A) Delivery of Drug to Affected Site

A drug (for example, an anticancer drug such as a low-molecular anticancer compound) can be locally delivered to an affected site (for example, cancer cells) by using the drug as the construct and altering the surface of the virus coat protein so as to exhibit affinity for the affected site.

### (B) Introduction of Nucleic Acid

The use of a nucleic acid as the construct enables the efficient introduction of the nucleic acid into cells. Examples of the nucleic acid can include functional nucleic acids (siRNA, miRNA, antisense nucleic acids, ribozymes, aptamers, and the like) in addition to foreign genes.

### (C) Delivery of Contrast Agent

A contrast agent (for example, ferrite) can be locally delivered to an affected site (for example, cancer cells) by using the drug as the construct and altering the surface of the virus coat protein so as to exhibit affinity for the affected site. This enables the visualization of the affected site with a T2-weighed image of MRI in vivo.

### (D) Probe in Biosensor

When a magnetic particle or the like is used as the construct, an antibody or the like can be modified by altering the surface of the virus coat protein and thereby can detect and measure an antigen or the like as a probe in a biosensor.

### (5) Method for Treating Disease

The method for treating a disease according to the present invention uses the above-described construct coated with a virus coat protein of the present invention and is a method for treating a disease in an animal, comprising administering a drug to the animal to deliver the drug to an affected site of the animal, wherein the drug to be administered is a drug to the surface of which a virus coat protein is attached to form a layer of the protein thereon.

The treatment method can be performed in the same manner as for a common drug delivery system except for coating a drug with a virus coat protein.

The disease to be treated is not particularly limited, and may be any of diseases to which a drug delivery system is generally applied, and the like. Specific examples thereof can include cancer, inflammatory diseases (pneumonia, hepatitis, cerebritis, arthritis, and the like), Alzheimer disease, diabetes, cerebral infarction, and myocardial infarction.

The drug is not particularly limited provided that it is a drug effective against any of the above diseases. Although a drug having a strong side effect, a drug for gene therapy, or the like is commonly used in a drug delivery system, such drugs may also be used in the present invention.

The animal to be treated may be a human and may be a non-human animal. Examples of the non-human animal can include domestic animals (for example, bovine, horse, porcine, and chicken), pet animals (for example, dog and cat), and experimental animals (mouse, rat, zebrafish, and the like).

According to the treatment method of the present invention, a substance having affinity for an affected site may be added to the virus coat protein so that the drug is specifically delivered to the affected site. Examples of the substance having affinity for an affected site can include an antibody or a ligand for a receptor on cells contained in the affected site.

This treatment method can be applied to a screening method for a new drug. Specifically, a candidate drug substance having a therapeutic effect can be selected through screening by administering the candidate drug substance to the surface of which a virus coat protein is attached to form a layer of the protein thereon, to a disease-model animal to deliver the drug to an affected site of the disease-model animal and then observing the disease state of the disease-model animal.

### Examples

The Examples described below are only preferable aspects within the scope of the present invention. The device names, numerical conditions such as the concentration, usage amount, treatment time, and treatment temperature of use materials, treatment methods, and the like used in Examples are only preferable examples within the scope of the present invention.

### Example 1: Preparation of Construct

Five constructs: polystyrene beads, silica beads, ferrite particles, a DOX-containing PLA spherical construct, and a siRNA-containing PLGA spherical construct were prepared or obtained as follows:

### (1) Polystyrene Bead

Three types of polystyrene beads having average particle diameters of 100 nm, 200 nm, and 500 nm were purchased from Polysciences, Inc. The purchased polystyrene beads (Cat # 16688-15, 08216-15, and 09836-15) were spherical and each assumes a negative charge because of having a carboxyl group.

### (2) Silica Bead

Silica beads having an average particle diameter of 110 nm were prepared. A method for preparing the same will be described below.

### A) Synthesis of Silica Bead

Tetraethoxysilane (4.5 ml) is dispersed in ethanol (83.5 ml), which is then stirred at 4°C for 30 minutes. Thereto is added 20 ml of a 2.8% ammonia aqueous solution cooled at 4°C in advance, which is then stirred a whole day and night. After the end of reaction, 100 ml of ultrapure water is added to the reaction solution, from which dissolved ethanol and ammonia are then removed using an evaporator; then, the silica bead dispersion solvent obtained by dialysis with ultrapure water is replaced by ultrapure water.

### B) Amination of Silica Bead

Using hydrochloric acid, 20 ml of ultrapure water in which 1 g of silica beads are dispersed is adjusted to a pH of 3 or less. To the silica bead dispersion is gently added a mixture of 1.8 ml of 3-aminopropyltrimethoxysilane as a silane coupling agent, 1.7 ml of 6N hydrochloric acid, and 2 ml of ultrapure water while stirring vigorously the dispersion. In addition, 25 ml of ethanol is added to the silica bead dispersion while continuing stirring. The silica bead dispersion is adjusted to a pH of 5.5 using a sodium hydroxide aqueous solution, which is then placed in a water bath at 70°C and continued to be stirred for 4 hours. The resultant aminated silica bead dispersion is transferred to a centrifuge tube and centrifuged at 20,000 G for 15 minutes, followed by discarding the supernatant. A pH 2 acetic acid solution (20 ml) is added to a precipitate of aminated silica beads to redisperse the particles, followed by centrifugation at 20,000 G for 15 minutes. This operation of washing the aminated beads with the acetic acid solution is repeated three times. The finally obtained precipitate of the aminated silica beads is re-dispersed in a pH 2 acetic acid solution and preserved.

The prepared silica beads are spherical and assume a positive charge because of having amino groups.

### (3) Ferrite Particle

Ferrite particles having an average particle diameter of 30 nm were prepared. A method for preparing the same will be described below.

### A) Synthesis of Ferrite Particle Precursor (Oleic Acid - Iron Complex)

Ferric chloride hexahydrate (10.78 g) is dissolved in ultrapure water (60 ml), to which sodium oleate (36.65 g) is then added. Thereto is added 80 ml of ethanol, which is then stirred at room temperature for 5 minutes. Thereafter, 140 ml of hexane is added thereto, which is then refluxed for 4 hours while stirring. After cooling the reaction solution, the aqueous phase is removed, followed by further washing the organic solvent phase with 30 ml of ultrapure water three times. After washing, the organic solvent phase is centrifuged at 238 G and 4°C for 10 minutes, and the supernatant is taken out and dried a whole day and night in an oven at 70°C.

### B) Synthesis of Ferrite Particle

The resultant ferrite particle precursor is dissolved in 190 g of tri-N-octylamine, to which 6.14 g of sodium oleate is then added, followed by stirring while increasing the temperature by 2°C every minute. After 100 minutes, it is heated to the boiling point of the solvent and refluxed for 30 minutes while stirring. After reaction, the resultant particles are washed 3 times with 30 ml of methanol and dispersed in 1-octadecene.

### C) Transfer of Ferrite Particle to Aqueous Phase

The resultant ferrite particles (180 mg) is washed 3 times with isopropanol (10 ml) and dispersed in toluene (16 ml) (in which dissolved oxygen is replaced by nitrogen). Thiomalic acid (0.3 g) is dissolved in dimethyl sulfoxide (4 ml) (in which dissolved oxygen is replaced by nitrogen), which is then added to the above toluene having ferrite particles dispersed; after charging nitrogen in the reaction vessel followed by sealing, the resultant is subjected to 35 kHz ultrasonication at 4°C for 4 hours. After ultrasonication, the ferrite particles are washed 5 times with 5 ml of 2-methoxyethanol, to which 10 ml of a 0.1 M citric acid aqueous solution of pH 7 is then added, followed by 35 kHz ultrasonication at 4°C for 1 hour. After reaction, 20 ml of 1,4-dioxane is added to the reaction solution to precipitate the ferrite particles, followed by discarding the supernatant. The particles is re-dispersed in 10 ml of ultrapure water and dialyzed in ultrapure water.

The prepared ferrite particles are cubical and assume a negative charge because of having citric acid.

### (4) DOX-Containing PLA Spherical Construct

A DOX-containing PLA spherical construct having an average particle diameter of around 200 nm was prepared. A method for preparing the same will be described below.

Ferrite particle precursor (2.5 mg), polylactic acid (Mw: 85 to 160 k) (12.5 mg), doxorubicin (1 mg), and triethylamine (10 equivalents relative to doxorubicin) are dissolved in chloroform (2 ml), which is used as an organic layer. Then, 2 ml of the organic layer is added dropwise to 6.25 ml of a 5% (wt/wt) PVA (Poly Vinyl Alcohol) aqueous solution under stirring, and the mixture is subjected to ultrasonic irradiation for 10 minutes to form a DOX-containing PLA spherical construct. The resultant construct is stirred overnight to remove chloroform, and centrifuged at 20,400 g for 10 minutes, followed by discarding the supernatant. Thereafter, an appropriate amount of ultrapure water is added thereto for re-dispersion, which is again centrifuged at 20,400 g for 10 minutes. The operation of washing with ultrapure water is repeated 3 times. Then, the construct is re-dispersed in ultrapure water and preserved.

### (5) siRNA-Containing PLGA Spherical Construct

A siRNA-containing PLGA spherical construct having an average particle diameter of around 200 nm was prepared. A method for preparing the same will be described below.

10 µl of an aqueous solution containing 1 to 10 µg of siRNA and 10 µl of an aqueous solution containing 0 to 100 µg of DOTAP (trimethyl[2,3-(dioleyloxy)propyl]-ammonium chloride) are mixed, which is then added to 400 µl of an acetone solution containing 10 mg of PLGA under stirring. Thereafter, the resultant is mixed with 5 ml of 2% (wt/wt) PVA under stirring. The resultant siRNA-containing PLGA spherical construct is centrifuged at 20,400 g for 10 minutes, followed by discarding the supernatant. Then, an appropriate amount of ultrapure water is added thereto for re-dispersion, which is again centrifuged at 20,400 g for 10 minutes. The operation of washing with ultrapure water is repeated 3 times. Thereafter, the construct is stirred in ultrapure water overnight to remove acetone, again washed with ultrapure water, and then preserved.

### Example 2: Coating Construct with Virus Coat Protein

The VP1 pentamer of SV40 was purified by a method as described in WO 2007/116808, and 10 µg of the purified protein was mixed with each construct prepared in Example 1. This mixed sample solution contained 150 mM NaCl, 5 mM EGTA, and 5 mM DTT, and the total volume was set at 100 µl.

The pH of the mixed sample solution was set at 5.0 (HCl [pH 5.0]) when the silica beads are used as a construct, at 7.0 (Tris-HCl [pH 7.0]) when the polystyrene beads and the ferrite particles were used, and at 7.9 (Tris-HCl [pH 7.9]) when the DOX-containing PLA spherical construct and the siRNA-containing PLGA spherical construct were used.

Then, the mixed sample solution was dialyzed with a solution containing 150 mM NaCl and 2 mM CaCl₂ at room temperature (25°C) for 10 hours to replace the solvent. The pH of the solution used for the dialysis is adjusted to the same pH as that of the mixed sample solution.

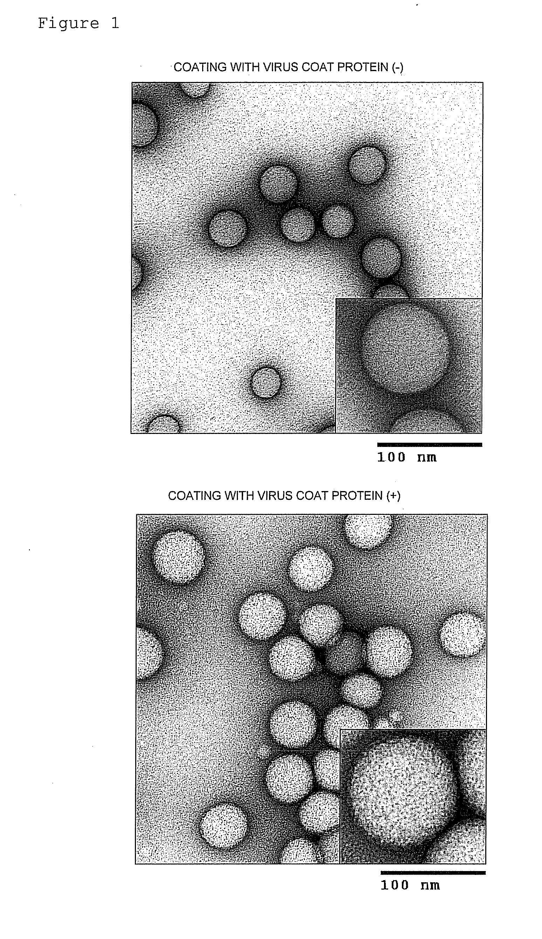

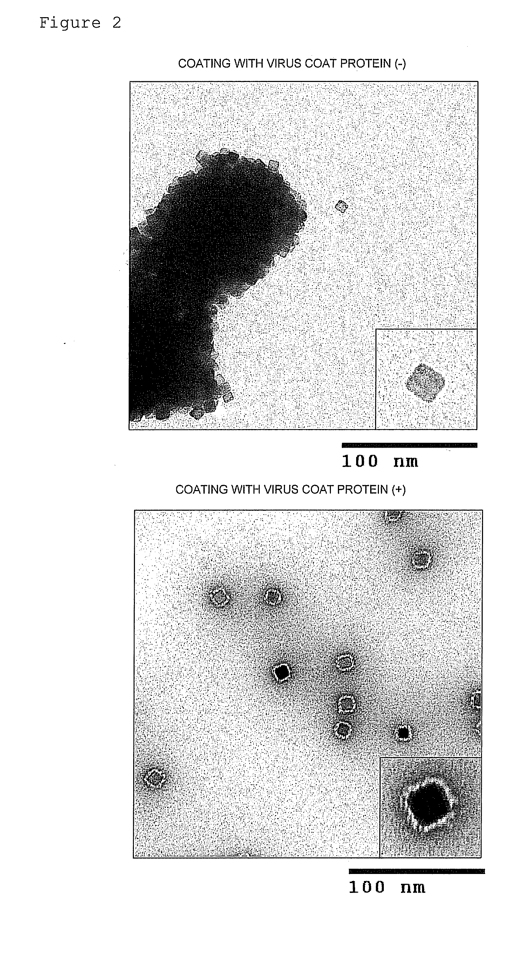

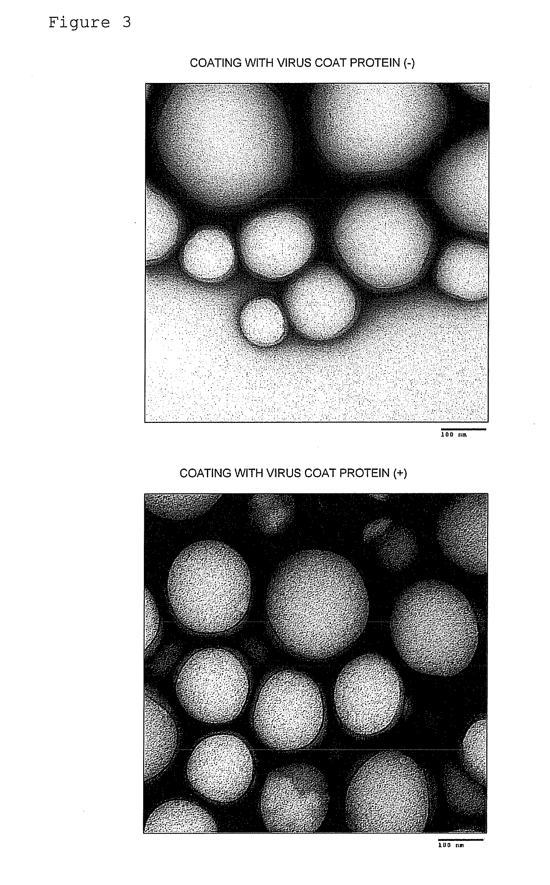

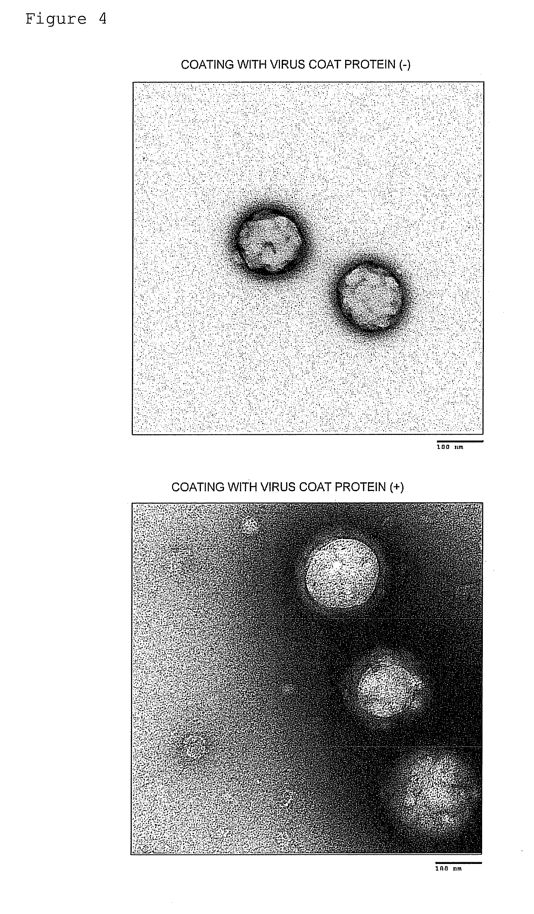

The mixed sample solution was recovered and the construct was observed using a transmission electron microscope. It was confirmed that the addition of the virus coat protein resulted in the construct surface being uniformly coated with the virus coat protein (Figures 1, 2, 3, and 4).

The above experiment showed that the virus coat protein could coat various constructs.

### Example 3: Staining of Construct Coated with Virus Coat Protein with Colloidal Gold

An anti-VP1 antibody and protein A-bound colloidal gold particles (10 nm) were used to verify coating with a virus coat protein. First, 4 µg of an IgG-purified anti-VP1 antibody was added to 1.85×10⁹ particles of the construct coated with a virus coat protein (polystyrene beads having an average particle diameter of 100 nm or silica beads having an average particle diameter of 110 nm) of Example 2, which was then incubated at 37°C for 1 hour. Thereafter, 1.85×10¹¹ particles of the colloidal gold were added thereto, which was then incubated at 37°C for 1 hour to perform colloidal gold staining. After reaction, the solution was preserved at 4°C.

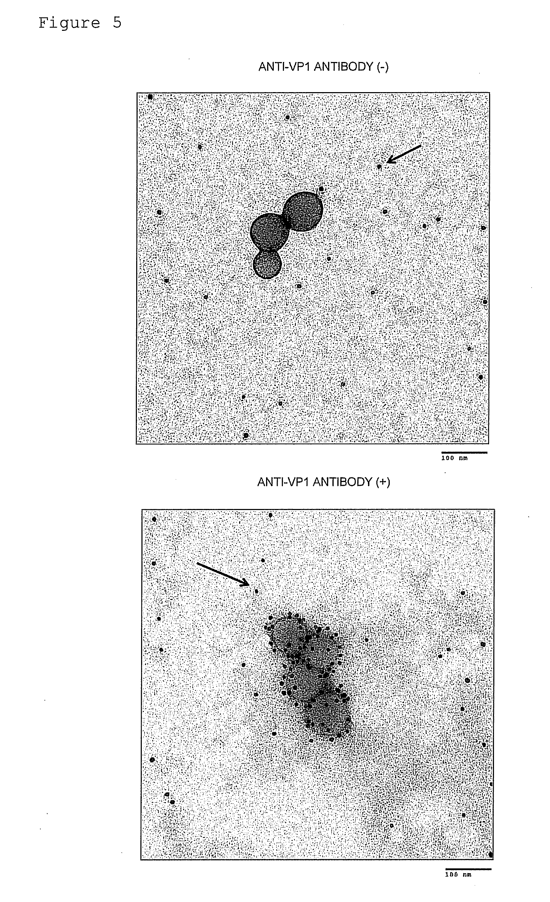

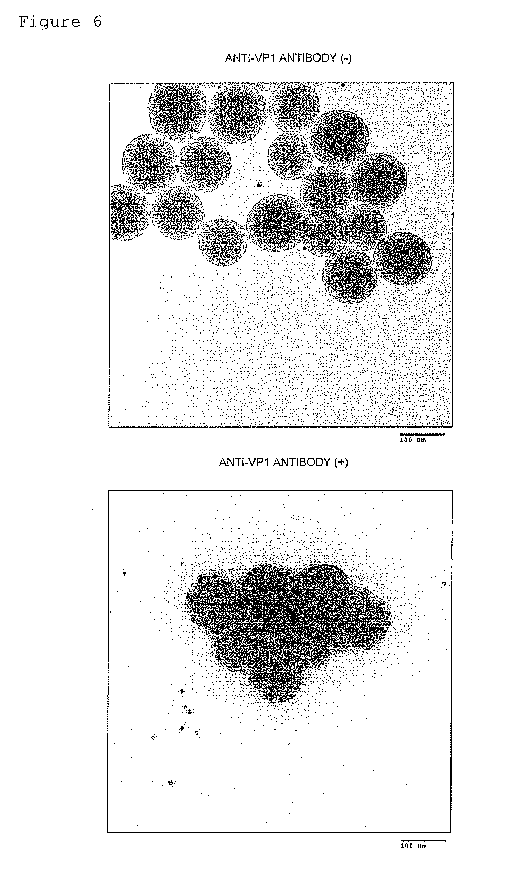

When the solution after reaction was observed under a transmission electron microscope, each of the polystyrene bead coated with the virus coat protein and the silica bead coated with the virus coat protein had no colloidal gold identified on the construct surface in the case of no addition of the anti-VP1 antibody (the top of Figure 5 and the top of Figure 6), while the colloidal gold was identified on the construct surface only in the case of the addition of the anti-VP1 antibody (the bottom of Figure 5 and the bottom of Figure 6).

These results showed that the surface of these constructs was coated with the virus coat protein. Example 4: Change in ζ Potential (Surface Potential) Due to Virus Coat Protein

The ζ potential of the construct coated with a virus coat protein (polystyrene beads having an average particle diameter of 100 nm, 200 nm, and 500 nm or silica beads having an average particle diameter of 110 nm) prepared in Example 2 and the construct not coated with a virus coat protein was measured. The measurement of the ζ potential was carried out under three conditions: pHs 5.0, 7.0 and 9.0.

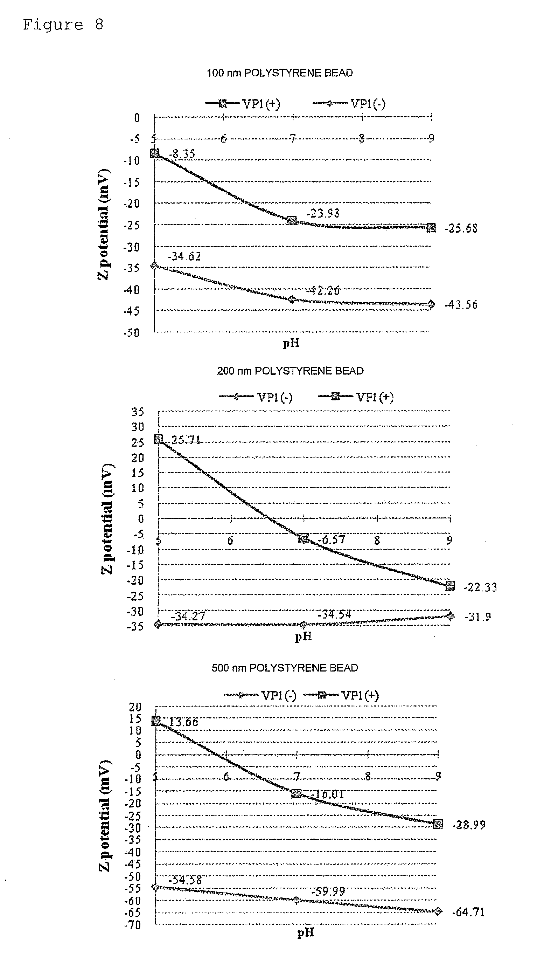

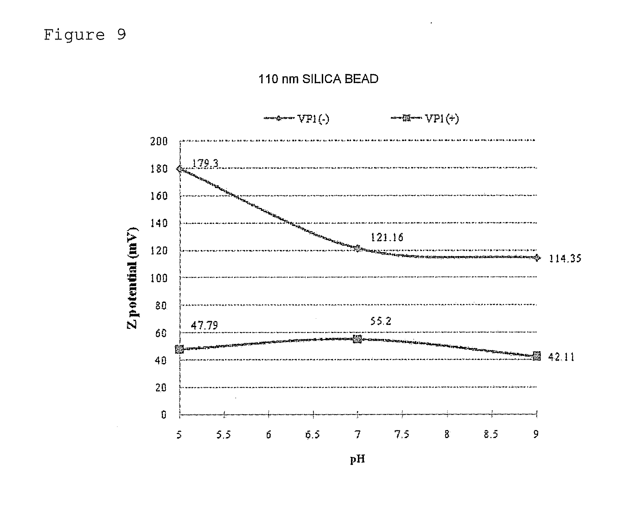

For both of the negatively charged construct (polystyrene beads) and the positively charged construct (silica beads), it was determined that coating with the virus coat protein resulted in the ζ potential of each construct approaching neutrality (Figures 8 and 9). This showed that the virus coat protein coated the construct surface.

### Example 5: Preparation of siRNA-Containing Liposome Coated with Virus Coat Protein

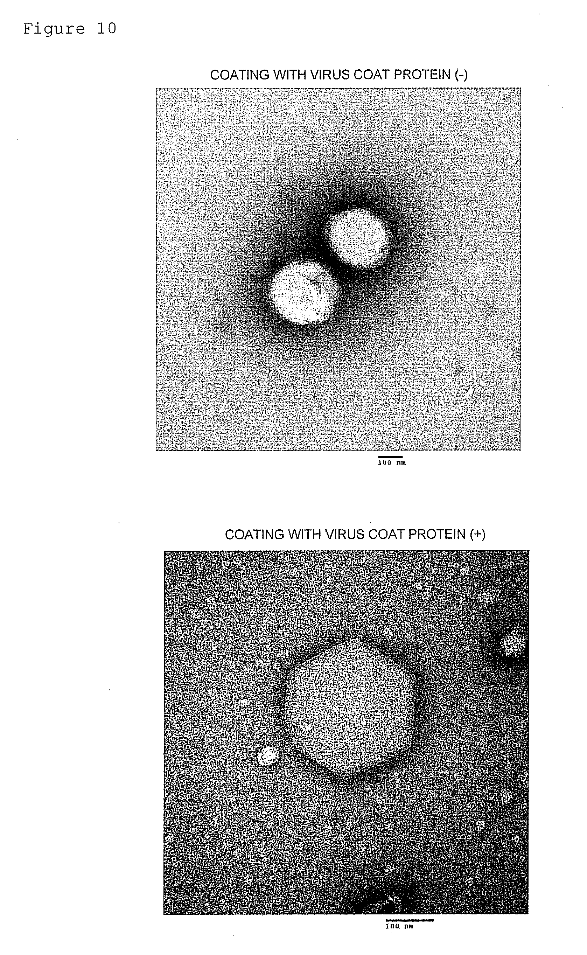

siRNA-containing liposomes coated with a virus coat protein were prepared according to methods as described in (1) to (3) below (Figure 10).

### (1) Preparation of Cationic Liposome

DOTAP and DOPE (molar ratio: 3:1) were mixed and diluted in chloroform to a lipid concentration of 5 mg/ml. To prepare a lipid membrane, the chloroform was evaporated from the mixture in a glass vial by a nitrogen stream. The membrane was re-hydrated in Tris-HCl buffer by intermittent stirring and then extruded through a polycarbonate filter (pore diameter: 100 nm) using Mini Extruder (from Avanti Polar Lipids, Inc.) to control the particle diameter of the resultant to about 100 nm.

### (2) Preparation of Liposome-siRNA Complex

siRNA (GL3) was mixed with the size-controlled liposomes (weight ratio: 1:10) to a lipid concentration of 455 µg/ml, followed by incubation at room temperature for 15 minutes.

### (3) Coating of siRNA-Containing Liposome with VP1

A VP1 pentamer was prepared by the same method as in Example 2. The VP1 pentamer (10 µg) was mixed with the liposome-siRNA complex (5 µg in lipid equivalent), which was then diluted with a buffer for preparing the VP1 pentamer (20 mM Tris-HCl pH 7.9, 150 mM NaCl, 5 mM DTT, 5 mM EGTA) to a volume of 100 µl, followed by overnight dialysis in a disposable polypropylene dialysis cup (Slide-A-Lyzer MINI Dialysis Unit, MWCO 3.5K, from Pierce) against a VLP construction buffer (20 mM MOPS pH 7, 150 mM NaCl).

### Reference Example: Active Targeting Using Ferrite-Containing Virus-Like Particle

Epidermal growth factor (EGF) was immobilized on ferrite-containing virus-like particles (VLP-Ferrite) as described in International Publication No. WO 2007/116808. How much a cell hyper-expressing an epidermal growth factor receptor (EGFR) ingested the EGF-immobilizing VLP-Ferrite was examined and the result was compared with the ingestion amount of EGF-unimmobilizing VLP-Ferrite.

Each of EGFR-hyper-expressing cells and EGFR-hypo-expressing cells was transplanted to mice, and the EGF-immobilizing VLP-Ferrite was administered to these mice to examine whether it was preferentially delivered to the target cells (EGFR-hyper-expressing cells).

### (1) Experimental Method

### (1-1) Preparation of N138C-Mutated VP1 Pentamer

VP1 (virus protein 1 of simian virus 40) gene was inserted into a vector, pFastBac. Asparagine as the 138th amino acid of VP1 was replaced by cysteine through site-directed mutagenesis. The plasmid was transformed into DH10BAC for the purpose of transfer to bacmid. The bacmid was subjected to lipofection into sf9 insect cells to express VP1-cloned baculovirus (P1 virus). The baculovirus infected sf9 cells and was incubated for 72 hours to increase the titer (P2 virus). To express VP1 protein, the prepared P2 baculovirus infected sf9 cells, and the infected cells were incubated for 72 hours. The resultant sf9 cells were re-suspended in ultrasonication buffer (1% sodium deoxycholate, 20 mM Tris-HCl, pH 7.9) in the presence of a protease inhibitor, and crushed on ice by ultrasonication. The insoluble fraction was removed from the crushed solution by centrifugation, and the supernatant was layered on a cesium chloride gradient (1.17, 1.28, 1.42, 1.58 g/ml in 20 mM Tris-HCl, pH 7.9) and centrifuged at 150,000xg for 3 hours in Beckman SW Ti 41 rotor. The VLP-containing fraction was re-suspended using cesium chloride to a final concentration of 1.38 g/ml and the suspension was centrifuged at 230,000xg for 20 hours in Beckman SW Ti 55 rotor.20. The VLP-containing fraction was dialyzed overnight against VLP suspension buffer (20 mM Tris-HCl, 150 mM NaCl, pH 7.9 0.1% NP40), and, after adding 5 mM DTT and EGTA to decompose VLP into pentamers, incubated at room temperature for 1 hour. The decomposed VP1 was further purified by gel filtration chromatography using HiLoad16/60Superdex200pg.

### (1-2) Preparation of Cross-Linker

A PEG cross-linker designated as NHS (PEO)₂ Mal was purchased from Pierce. This cross-linker has succinimide at one end and maleimide at the other end. The cross-linker is diluted to a concentration of 261.1 mM with dry DMSO and preserved at -30°C.

### (1-3) Preparation of EGF

A recombinant hEGF (human epidermal growth factor) protein was purchased from Peprotech. The hEGF was diluted with water to a concentration of 2.5 mg/ml, and then dialyzed against MOPS buffer (20 mM MOPS, pH 6.5, 150 mM NaCl) for 8 hours. The protein concentration was evaluated by Bradford assay using BSA for calibration and then the EGF solution was preserved at -20°C.

### (1-4) Immobilization of EGF on VLP-Ferrite

The cross-linker in DMSO was diluted with a 100-fold volume of MOPS buffer (20 mM MOPS, 150 mM NaCl pH 6.5, the same as the buffer for EGF). The hEGF was mixed with the diluted cross-linker (molar ratio: 1:1) and incubated at 4°C for 2 hours. After incubation, ethanolamine buffered at pH 6.5 by MOPS was added to a concentration of 100 mM to stop the reaction, and dialyzed overnight against the same buffer to remove the unbound cross-linker. On the next day, the dialyzed sample was collected and mixed with VLP-ferrite to a molar ratio of 3,000:1. After 12 hours of reaction, NaOH was added to stop the reaction. The unbound hEGF or the hEGF bound to the cross-linker were removed by centrifugation twice in PBS at 15,000 rpm for 15 minutes.

### (1-5) In Vitro Ingestion of VLP-Ferrite

4×10⁵ CV1, A431 or WiDR cells were seeded on a culture dish containing DMEM supplemented with 10% fetal bovine serum and incubated overnight under conditions of 37°C and 5% CO₂. Cells were made into a starvation state by incubation in DMEM free of serum for 30 minutes under conditions of 37°C and 5% CO₂. VLP-ferrite (30 µg in iron equivalent) on which EGF is immobilized or not immobilized was added to cells, followed by incubation under conditions of 37°C and 5% CO₂ for 1 hour. 10% FBS was added to the medium, followed by further incubation for 11 hours. Cells were washed twice with PBS, scraped, and then transferred to an Eppendorf tube. To obtain a cell pellet, the suspension was centrifuged at 15,000 rpm and 4°C for 1 minute. The pellet was re-suspended in PBS and subjected to ultrasonication to lyse cells, and nitric acid was added thereto to decompose the ferrite (magnetite) into iron ion. The solution containing iron ion was standardized using yttrium, and subjected to ICP-OES analysis to measure the iron content.

### (1-6) In Vivo Imaging of VLP-Ferrite by MRI

The active targeting and imaging of VLP-ferrite were monitored by in vivo MRI. Mice were subjected to the transplantation of A431 and WiDR into the left and right sides, respectively, thereof. VLP-ferrite having EGF immobilized (1 mg in iron equivalent) (about 50 mg/kg) was intravenously administered. Imaging of MRI was carried out using 7T MRI before and after injection.

### (2) Experimental Result

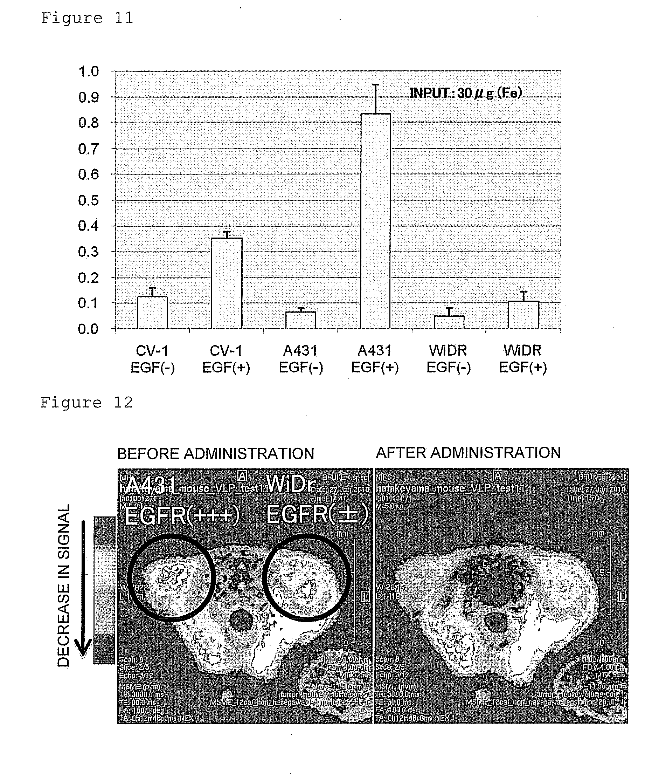

The capabilities of various cells to ingest VLP-ferrite having EGF immobilized and VLP-ferrite having no EGF immobilized are shown in Figure 11. As shown in the figure, for A431 as an EGFR-hyper-expressing cell, the immobilization of EGF significantly enhanced the ingesting capability thereof. CV-1 as a host for SV-40 and WiDr as an EGFR-hypo-expressing cell also showed the enhanced ingesting capabilities thereof due to the immobilization of EGF.

MRI images before and after administering VLP-ferrite are shown in Figure 12. As shown in the figure, the signal from the site having A431 transplanted was markedly decreased after administrating VLP-ferrite. Because VLP-ferrite has a contrast effect, it is probable that the contrast effect decreased the signal from the site having A431 transplanted since VLP-ferrite was preferentially delivered to A431.

The above results are those obtained using the virus-like particles (VLP-ferrite) described in International Publication No. WO 2007/116808 and not those obtained using the construct of the present invention. However, because like the virus-like particle, the construct of the present invention can also immobilize a protein such as EGF, it can probably specifically deliver the protein to target cells.

### Industrial Applicability

The present invention facilitates the delivery of a drug to an affected site, and is useful for the development of a new drug delivery system.

## Claims

1. A construct coated with a virus coat protein, comprising a virus coat protein and a construct coated therewith, wherein the virus coat protein is attached to the surface of the construct to form a layer of the protein thereon.

2. The construct coated with a virus coat protein according to claim 1, wherein the virus coat protein is non-specifically attached to the surface of the construct.

3. The construct coated with a virus coat protein according to claim 1 or 2, wherein the construct is a construct having such a large size that it can not be packaged in a virus particle.

4. The construct coated with a virus coat protein according to claim 1 or 2, wherein the construct has a particle diameter of 30 nm or more.

5. The construct coated with a virus coat protein according to any one of claims 1 to 4, wherein the virus coat protein is a coat protein of SV40 virus.

6. A method for producing a construct coated with a virus coat protein, comprising incubating the virus coat protein and the construct at 15 to 30°C and pH 5 to pH 10 in the presence of a 100 mM to 500 mM monovalent cation and a 2 µM to 50 mM divalent cation, wherein the construct is a construct having such a large size that it can not be packaged in a virus particle.

7. The method for producing a construct coated with a virus coat protein according to claim 6, wherein the construct has a particle diameter of 30 nm or more.

8. The method for producing a construct coated with a virus coat protein according to claim 6 or 7, wherein the virus coat protein is a coat protein of SV40 virus.

9. A method for treating a disease in an animal, comprising administering a drug to the animal to deliver the drug to an affected site of the animal, wherein the drug to be administered is a drug to the surface of which a virus coat protein is attached to form a layer of the protein thereon.

10. The method for treating a disease according to claim 9, wherein the animal is a non-human animal.

11. The method for treating a disease according to claim 9 or 10, wherein a substance having affinity for the affected site is added to the virus coat protein.

12. The method for treating a disease according to any one of claims 9 to 11, wherein the virus coat protein is non-specifically attached to the surface of the drug.

13. The method for treating a disease according to any one of claims 9 to 12, wherein the drug is a drug which is large enough not to be packaged in a virus particle.

14. The method for treating a disease according to any one of claims 9 to 13, wherein the drug has a particle diameter of 30 nm or more.

15. The method for treating a disease according to any one of claims 9 to 14, wherein the virus coat protein is a coat protein of SV40 virus.
